# EUROPEAN PATENT SPECIFICATION

(11) **EP 3 820 892 B1**
(45) Date of publication and mention of the grant of the patent: **03.01.2024**
(21) Application number: 19735588.6
(22) Date of filing: 09.07.2019
(51) Int. Cl.: C07K 16/10, G01N 33/576

(54) **ANTIBODIES HAVING SPECIFICITY FOR THE ORF2I PROTEIN OF HEPATITIS E VIRUS AND USES THEREOF FOR DIAGNOSTIC PURPOSES**
ANTIKÖRPER MIT SPEZIFITÄT FÜR DAS ORF2I-PROTEIN DES HEPATITIS-E-VIRUS UND VERWENDUNGEN DAVON FÜR DIAGNOSTISCHE ZWECKE
ANTICORPS PRÉSENTANT UNE SPÉCIFICITÉ POUR LA PROTÉINE ORF2I DU VIRUS DE L'HÉPATITE E ET LEURS UTILISATIONS À DES FINS DE DIAGNOSTIC

(30) Priority: 10.07.2018 EP 18305918
(43) Date of publication of application: 19.05.2021
(73) Proprietor: Institut National de la Santé et de la Recherche Médicale (INSERM), 75013 Paris (FR); Institut Pasteur de Lille, 59000 Lille (FR); Centre National de la Recherche Scientifique (CNRS), 75016 Paris (FR); Université de Lille, 59800 Lille (FR)
(72) Inventor: COCQUEREL-DEPROY, Laurence, 59021 Lille cedex (FR); MONTPELLIER, Claire, 59021 Lille cedex (FR); DUBUISSON, Jean, 59021 Lille cedex (FR)
(74) Representative: Inserm Transfert
(86) International application number: PCT/EP2019/068338
(87) International publication number: WO 2020/011752

(56) References cited:
- MONTPELLIER CLAIRE ET AL: "Hepatitis E Virus Lifecycle and Identification of 3 Forms of the ORF2 Capsid Protein", GASTROENTEROLOGY, W.B. SAUNDERS CO, US, vol. 154, no. 1, 25 September 2017 (2017-09-25), page 211, XP085311735, ISSN: 0016-5085, DOI: 10.1053/J.GASTRO.2017.09.020
- KOBAYASHI TOMINARI ET AL: "Characterization and epitope mapping of monoclonal antibodies raised against rat hepatitis E virus capsid protein: An evaluation of their neutralizing activity in a cell culture system", JOURNAL OF VIROLOGICAL METHODS, ELSEVIER BV, NL, vol. 233, 16 March 2016 (2016-03-16), pages 78-88, XP029511837, ISSN: 0166-0934, DOI: 10.1016/J.JVIROMET.2016.03.004

## Description

### FIELD OF THE PRESENT INVENTION:

The present invention relates to antibodies having specificity for the ORF2i protein of hepatitis E virus and uses thereof for diagnostic purposes.

### BACKGROUND OF THE PRESENT INVENTION:

Hepatitis E virus (HEV) is annually responsible for 20 million infections with 3.4 million symptomatic cases and 70,000 deaths mainly occurring in less developed regions of the world. It causes acute hepatitis either as water-borne outbreaks or as sporadic cases through the fecal-oral route (Debing Y, Moradpour D, Neyts J, et al. Update on Hepatitis E Virology:Implications for Clinical Practice. Journal of Hepatology 2016;65:200-212*).* Though infection by HEV is usually self-resolving, severe forms or chronic infections have been described, mainly in immunocompromised patients. A high rate of mortality has also been reported among pregnant women. HEV infection has also been associated with extrahepatic disorders (Pischke S, Hartl J, Pas SD, et al. Hepatitis E virus infection beyond the liver? Journal of Hepatology 2016. 10.1016/j.jhep.2016.11.016*).* Four genotypes (gt) are pathogenic in humans. Gtl and gt2 exclusively infect humans, whereas gt3 and gt4 are zoonotic and mainly infect mammalian animals with occasional transmission to humans. Recently, gt3 infections have been emerging in the Western world likely due to the consumption of contaminated food and blood transfusion (Debing Y, Moradpour D, Neyts J, et al. Update on Hepatitis E Virology:Implications for Clinical Practice. Journal of Hepatology 2016;65:200-212*).* The diagnosis of hepatitis E is based on detecting anti-HEV antibodies and/or viral RNA in patient serum (Khuroo MS, Khuroo MS. Hepatitis E: an emerging global disease - from discovery towards control and cure. Journal of Viral Hepatitis 2016;23:68-79)*.* Recently, a new assay based on detection of the HEV antigen capsid protein was developed (Wantaï Biologicals) notably for laboratories with no molecular diagnosis facilities.

HEV is a quasi-enveloped virus containing a linear, single-stranded, positive-sense RNA genome that contains three open reading frames (ORFs), namely, ORF1, ORF2 and ORF3 (Debing Y, Moradpour D, Neyts J, et al. Update on Hepatitis E Virology:Implications for Clinical Practice. Journal of Hepatology 2016;65:200-212)*.* ORF1 is the largest gene that encodes a non-structural polyprotein (ORF1 protein) that contains several functional domains essential for viral replication. ORF2 encodes the ORF2 viral capsid protein, which is involved
in particle assembly, binding to host cells and eliciting neutralizing antibodies. ORF3 encodes a small multifunctional phosphoprotein that is involved in virion morphogenesis and egress. Although HEV is a non-enveloped virus in bile and feces, patient serum and cell culture-produced particles have been described to be associated with cellular lipids and display the ORF3 protein at their surface (Okamoto H. Culture systems for hepatitis E virus. J Gastroenterol 2012;48:147158)*.*

Recently, 3 different forms of the ORF2 capsid protein were identified: infectious/intracellular ORF2 (ORF2i), glycosylated ORF2 (ORF2g), and cleaved ORF2 (ORF2c) (Montpellier C., et al. "Hepatitis E virus lifecycle and identification of 3 forms of the ORF2 capsid protein." Gastroenterology 154.1 (2018): 211-223)*.* The ORF2i protein, for which the precise sequence has been identified, is the form that is associated with infectious particles. The ORF2i protein is not glycosylated and is likely not translocated into the endoplasmic reticulum (ER) lumen and stays in the cytosolic compartment. In contrast, ORF2g and ORF2c proteins are secreted in large amounts in cell culture and infected patient sera, sialylated, N- and O-glycosylated but are not associated with infectious virions. Importantly, ORF2g and ORF2c proteins are the main antigens present in HEV-infected patient sera. Accordingly antibodies having specificity for the ORF2i protein would be suitable for the diagnosis of HEV.

### SUMMARY OF THE PRESENT INVENTION:

The present invention relates to antibodies having specificity for the ORF2i protein of hepatitis E virus and uses thereof for diagnostic purposes. In particular, the present invention is defined by the claims.

### DETAILED DESCRIPTION OF THE PRESENT INVENTION:

The first object of the present invention relates to an antibody which binds to the ORF2i protein of hepatitis E virus and wherein said antibody does not bind to the ORF2g protein nor to the ORF2c protein of hepatitis E virus, and wherein the epitope of said antibody comprises at least one amino acid residue from amino acid residues 10 to 23 of SEQ ID NO: 1.

The terms "peptide", "polypeptide", and "protein" are used interchangeably, and refer to a compound comprised of amino acid residues covalently linked by peptide bonds. A polypeptide is not limited to a specific length: it must contain at least two amino acids, and no limitation is placed on the maximum number of amino acids that can comprise a polypeptide's sequence. Peptides, oligopeptides, and proteins are included within the definition of polypeptide, and such terms may be used interchangeably herein unless specifically indicated otherwise. As used herein, the term refers to both short chains, which also commonly are referred to in the art as peptides, oligopeptides and oligomers, for example, and to longer chains, which generally are referred to in the art as proteins, of which there are many types. In one embodiment, as used herein, the term "peptides" refers to a linear polymer of amino acids linked together by peptide bonds, preferably having a chain length of less than about 50 amino acids residues; a "polypeptide" refers to a linear polymer of at least 50 amino acids linked together by peptide bonds; and a protein specifically refers to a functional entity formed of one or more peptides or polypeptides, optionally glycosylated, and optionally of non-polypeptides cofactors. This term also does exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

As used herein, the term "ORF2i protein" refers to the hepatitis E virus ORF2 capsid protein (ORF2i) comprising an amino acid sequence having at least 90% of identity with the amino acid sequence as set forth in SEQ ID NO:1 and wherein said protein is not glycosylated.

SEQ ID NO:1:
1LPMLPAPPA10GQPSGRRRGRRSGG23AGGGFWGDRVDSQPFALPYIHPTNPFAADIVSQSGAG
TRPRQPPRPLGSAWRDQSQRPSAAPRRRSAPAGAAPLTAVSPAPDTAPVPDVDSRGAILRRQYN
LSTSPLTSSVASGTNLVLYAAPLNPLLPLQDGTNTHIMATEASNYAQYRVVRATIRYRPLVPNA
VGGYAISISFWPQTTTTPTSVDMNSITSTDVRILVQPGIASELVIPSERLHYRNQGWRSVETTGV
AEEEATSGLVMLCIHGSPVNSYTNTPYTGALGLLDFALELEFRNLTPGNTNTRVSRYTSTARHR
LRRGADGTAELTTTAATRFMKDLHFTGTNGVGEVGRGIALTLFNLADTLLGGLPTELISSAGG
QLFYSRPVVSANGEPTVKLYTSVENAQQDKGITIPHDIDLGDSRVVIQDYDNQHEQDRPTPSPA
PSRPFSVLRANDVLWLSLTAAEYDQATYGSSTNPMYVSDTVTFVNVATGAQAVARSLDWSKV
TLDGRPLTTIQQYSKTFYVLPLRGKLSFWEAGTTRAGYPYNYNTTASDQILIENAAGHRVAIST
YTTSLGAGPASISAVGVLAPHSALAVLEDTVDYPARAHTFDDFCPECRTLGLQGCAFQSTIAEL
QRLKTEVGKTRES

According to the invention, the mass of the ORF2i protein of the present invention is approximately 80kDa.

As used herein, the term "ORF2g protein" refers to the hepatitis E virus ORF2 capsid protein (ORF2g) comprising an amino acid sequence having at least 90% of identity with the amino acid sequence as set forth in SEQ ID NO:2 and wherein said protein is glycosylated.

SEQ ID NO:2:
SGGAGGGFWGDRVDSQPFALPYIHPTNPFAADIVSQSGAGTRPRQPPRPLGSAWRDQSQRPSA
APRRRSAPAGAAPLTAVSPAPDTAPVPDVDSRGAILRRQYNLSTSPLTSSVASGTNLVLYAAPL
NPLLPLQDGTNTHIMATEASNYAQYRVVRATIRYRPLVPNAVGGYAISISFWPQTTTTPTSVDM
NSITSTDVRILVQPGIASELVIPSERLHYRNQGWRSVETTGVAEEEATSGLVMLCIHGSPVNSYT
NTPYTGALGLLDFALELEFRNLTPGNTNTRVSRYTSTARHRLRRGADGTAELTTTAATRFMKD
LHFTGTNGVGEVGRGIALTLFNLADTLLGGLPTELISSAGGQLFYSRPVVSANGEPTVKLYTSV
ENAQQDKGITIPHDIDLGDSRVVIQDYDNQHEQDRPTPSPAPSRPFSVLRANDVLWLSLTAAEY
DQATYGSSTNPMYVSDTVTFVNVATGAQAVARSLDWSKVTLDGRPLTTIQQYSKTFYVLPLR
GKLSFWEAGTTRAGYPYNYNTTASDQILIENAAGHRVAISTYTTSLGAGPASISAVGVLAPHSA
LAVLEDTVDYPARAHTFDDFCPECRTLGLQGCAFQSTIAELQRLKTEVGKTRES

According to the invention, the mass of the ORF2g protein of the present invention is approximately 90kDa.

As used herein, the term "ORF2c protein" refers to the hepatitis E virus ORF2 capsid protein (ORF2c) comprising an amino acid sequence having at least 90% of identity with the amino acid sequence as set forth in SEQ ID NO:3 and wherein said protein is glycosylated.

SEQ ID NO: 3
SAPAGAAPLTAVSPAPDTAPVPDVDSRGAILRRQYNLSTSPLTSSVASGTNLVLYAAPLNPLLPL
QDGTNTHIMATEASNYAQYRVVRATIRYRPLVPNAVGGYAISISFWPQTTTTPTSVDMNSITST
DVRILVQPGIASELVIPSERLHYRNQGWRSVETTGVAEEEATSGLVMLCIHGSPVNSYTNTPYT
GALGLLDFALELEFRNLTPGNTNTRVSRYTSTARHRLRRGADGTAELTTTAATRFMKDLHFTG
TNGVGEVGRGIALTLFNLADTLLGGLPTELISSAGGQLFYSRPVVSANGEPTVKLYTSVENAQQ
DKGITIPHDIDLGDSRVVIQDYDNQHEQDRPTPSPAPSRPFSVLRANDVLWLSLTAAEYDQATY
GSSTNPMYVSDTVTFVNVATGAQAVARSLDWSKVTLDGRPLTTIQQYSKTFYVLPLRGKLSF
WEAGTTRAGYPYNYNTTASDQILIENAAGHRVAISTYTTSLGAGPASISAVGVLAPHSALAVLE
DTVDYPARAHTFDDFCPECRTLGLQGCAFQSTIAELQRLKTEVGKTRES

According to the invention, the mass of the ORF2c protein of the present invention is approximately 75kDa.

As used herein, the term "glycosylated" with respect to a protein means that a carbohydrate moiety is present at one or more sites of the protein molecule. In particular, a glycosylated protein refers to a protein that is typically modified by N-glycan or O-glycan addition.

According to the invention a first amino acid sequence having at least 90% of identity with a second amino acid sequence means that the first sequence has 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890,1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program^{®} 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

As used herein, the term "antibody" has its general meaning in the art and refers to any antibody-like molecule that has an antigen binding region, and this term includes antibody fragments that comprise an antigen binding domain such as Fab', Fab, F(ab')2, and single domain antibodies (DABs). In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CHl, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1 , L-CDR2, L- CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

The term "antibody fragment" refers to at least one portion of an intact antibody, preferably the antigen binding region or variable region of the intact antibody, that retains the ability to specifically interact with (*e.g.,* by binding, steric hindrance, stabilizing/destabilizing, spatial distribution) an epitope of an antigen. Examples of antibody fragments include, but are not limited to, Fab, Fab', F(ab')₂, Fv fragments, single chain antibody molecules, in particular scFv antibody fragments, disulfide-linked Fvs (sdFv), a Fd fragment consisting of the VH and CHI domains, linear antibodies, single domain antibodies such as, for example, sdAb (either VL or VH), camelid VHH domains, multi-specific antibodies formed from antibody fragments such as, for example, a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, *e.g*., Hollinger and Hudson, Nature Biotechnology 23:1126-1136, 2005). Antigen binding fragments can also be grafted into scaffolds based on polypeptides such as a fibronectin type III (see U.S. Patent No.: 6,703,199, which describes fibronectin polypeptide minibodies). Papain digestion of antibodies produces two identical antigen-binding fragments, called "Fab" fragments, and a residual "Fc" fragment, a designation reflecting the ability to crystallize readily.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab' " refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH:VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

According to the present invention, the antibody of the present invention has specificity for the ORF2i protein. As used herein, the term "specificity" refers to the ability of an antibody to detectably bind to an epitope presented on the ORF2i protein, while having relatively little detectable reactivity with the ORF2g protein and the ORF2c protein.

As used herein, the term "epitope" refers to a specific arrangement of amino acids located on a protein to which an antibody binds. Epitopes often consist of a chemically active surface grouping of molecules such as amino acids or sugar side chains, and have specific three dimensional structural characteristics as well as specific charge characteristics. Epitopes can be linear or conformational, *i.e.,* involving two or more sequences of amino acids in various regions of the antigen that may not necessarily be contiguous. The epitopes of the present invention thus comprise at least one amino acid residue from amino acid residues 10 to 23 of SEQ ID NO: 1 (i.e. SEQ ID NO:4).
SEQ ID NO:4
GOPSGRRRGRRSGG

In some embodiments, the antibody of the disclosure which is not part of the invention binds to an epitope comprising 1, 2, 3, 4, 5, 6, 7, 8, 9, 10, 11, 12, 13, or 14 amino acid residues from SEQ ID NO:4, or from a sequence sharing at least 90% of identity over SEQ ID NO: 4.

The antibody of the invention binds to an epitope comprising the amino acid sequence as set forth in SEQ ID NO: 4. An antibody of the disclosure which not part of the invention binds an amino acid sequence sharing at least 90% of identity over SEQ ID NO: 4.

As used herein, the term "binding" as used herein refers to a direct association between two molecules, due to, for example, covalent, electrostatic, hydrophobic, and ionic and/or hydrogen-bond interactions, including interactions such as salt bridges and water bridges. In particular, as used herein, the term "binding" in the context of the binding of an antibody to a predetermined antigen or epitope typically is a binding with an affinity corresponding to a K_{D} of about 10⁻⁷ M or less, such as about 10⁻⁸ M or less, such as about 10⁻⁹ M or less, about 10⁻¹⁰ M or less, or about 10⁻¹¹ M or even less.

Specificity can be relatively determined by binding or competitive binding assays, using, e.g., Biacore instruments, as described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules. The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One preferred and standard method well known in the art for determining the affinity of mAbs is the use of Biacore instruments.

In some embodiments, the antibody is a polyclonal antibody or a monoclonal antibody. The term "polyclonal antibody" as used herein refers to multiple immunoglobulins in antiserum produced to an antigen following immunization, and which may recognize and bind to one or more epitopes to that antigen. The terms "monoclonal antibody", "monoclonal Ab", "monoclonal antibody composition", "mAb", or the like, as used herein refer to a preparation of antibody molecules of single molecular composition. Monoclonal antibodies may be generated using the method of Kohler and Milstein (Nature, 256:495, 1975). To prepare monoclonal antibodies useful in the invention, a mouse or other appropriate host animal (e.g. mouse, goat, camelid...) is immunized at suitable intervals (e.g., twice-weekly, weekly, twice-monthly or monthly) with a relevant viral antigenic form. Typically, the immunogenic form consists of the peptide having the amino acid sequence ranging from the amino residue at position 10 to 23 in SEQ ID NO: 1 (i.e. SEQ ID NO:4). The animal may be administered a final "boost" of the antigenic form within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well- known in the field. The animals may be immunized by subcutaneous, intraperitoneal, intramuscular, intravenous, intranasal or other routes. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, immunofluorescence, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

In some embodiments, the antibody of the present invention is conjugated with a detectable label. Suitable detectable labels include, for example, a radioisotope, a fluorescent label, a chemiluminescent label, an enzyme label, a bio luminescent label or colloidal gold. Methods of making and detecting such detectably-labeled immunoconjugates are well-known to those of ordinary skill in the art, and are described in more detail below. For instance, the detectable label can be a radioisotope that is detected by autoradiography. Isotopes that are particularly useful for the purpose of the present invention are 3H, 125I, 131I, 35S and 14C. The antibody of the present invention can also be labeled with a fluorescent compound. The presence of a fluorescently-labeled antibody of the present invention is determined by exposing the immuno conjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine and Alexa Fluor dyes. Alternatively, the antibody of the present invention can be detectably labeled by coupling said antibody to a chemiluminescent compound. The presence of the chemiluminescent-tagged immuno conjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester. Similarly, a bio luminescent compound can be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin. Typically, when the antibody is conjugated to an enzyme then, the antibody is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase and alkaline phosphatase. Those of skill in the art will know of other suitable labels which can be employed in accordance with the present invention. The binding of marker moieties to anti-the antibody of the present invention is accomplished using standard techniques known to the art. Typical methodology in this regard is described by Kennedy et al., Clin. Chim. Acta 70: 1, 1976; Schurs et al., Clin. Chim. Acta 81 : 1, 1977; Shih et al., Int'U. Cancer 46: 1101, 1990; Stein et al, Cancer Res. 50: 1330, 1990; and Coligan, supra. Moreover, the convenience and versatility of immunochemical detection can be enhanced by using antibodies of the present invention that have been conjugated with avidin, streptavidin, and biotin. {See, e.g., Wilchek et al. (eds.), "Avidin-Biotin Technology," Methods In Enzymology (Vol. 184) (Academic Press 1990); Bayer et al., "Immunochemical Applications of Avidin-Biotin Technology," in Methods In Molecular Biology (Vol. 10) 149-162 (Manson, ed., The Humana Press, Inc. 1992).).

In some embodiments, the antibody of the present invention is conjugated with a detectable label. Suitable detectable labels include, for example, a radioisotope, a fluorescent label, a chemiluminescent label, an enzyme label, a bio luminescent label or colloidal gold. Methods of making and detecting such detectably-labeled immunoconjugates are well-known to those of ordinary skill in the art, and are described in more detail below. For instance, the detectable label can be a radioisotope that is detected by autoradiography. Isotopes that are particularly useful for the purpose of the present invention are ³H, ¹²⁵I, ¹³¹I, ³⁵S and ¹⁴C. The
antibody of the present invention can also be labeled with a fluorescent compound. The presence of a fluorescently-labeled antibody of the present invention is determined by exposing the immuno conjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine and Alexa Fluor dyes. Alternatively, the antibody of the present invention can be detectably labeled by coupling said antibody to a chemiluminescent compound. The presence of the chemiluminescent-tagged immuno conjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester. Similarly, a bio luminescent compound can be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin. Typically, when the antibody is conjugated to a fluorescent label as described above, the presence of the fusion protein can be detected with any means well known in the art such as a microscope or microscope or automated analysis system. Typically, when antibody is conjugated to an enzyme then, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase (e.g. horseradish peroxidase) and alkaline phosphatase. Those of skill in the art will know of other suitable labels which can be employed in accordance with the present invention. The binding of marker moieties to anti-the antibody of the present invention is accomplished using standard techniques known to the art. Typical methodology in this regard is described by Kennedy et al., Clin. Chim. Acta 70: 1, 1976; Schurs et al., Clin. Chim. Acta 81 : 1, 1977; Shih et al., Int'U. Cancer 46: 1101, 1990; Stein et al, Cancer Res. 50: 1330, 1990; and Coligan, supra. Moreover, the convenience and versatility of immunochemical detection can be enhanced by using antibodies of the present invention that have been conjugated with avidin, streptavidin, and biotin. {See, e.g., Wilchek et al. (eds.), "Avidin-Biotin Technology," Methods In Enzymology (Vol. 184) (Academic Press 1990); Bayer et al., "Immunochemical Applications of Avidin-Biotin Technology," in Methods In Molecular Biology (Vol. 10) 149- 162 (Manson, ed., The Humana Press, Inc. 1992).).

The antibodies of the present invention are particularly of interest for diagnostic purposes. In particular, the antibodies of the present invention are suitable for determining presence of infectious particles of hepatitis E virus in a sample. More particularly, the antibodies of the present invention are suitable for diagnosing hepatitis E virus infection in a subject.

Accordingly a further object of the present invention relates to a method for detecting the presence of the ORF2i protein in a sample comprising contacting the sample with the antibodies of the present invention under conditions that allow an immunocomplex of the protein and the antibodies to form wherein detection of the immunocomplex indicates the presence of the ORF2i protein in the sample (for instance: immunoprecipitation, immunofluorescence and western blotting).

As used herein, the term "sample" includes any solid or fluid sample, liable to contain infectious particles of hepatitis E virus. In some embodiments, the sample is selected from the group consisting of ascites; urine; saliva; sweat; milk; synovial fluid; peritoneal fluid; amniotic fluid; cerebrospinal fluid; lymph fluid; lung embolism; and pericardial fluid. In some embodiments, the sample is a faeces samples. In some embodiments, the sample is a urine sample. In some embodiments, the sample is a saliva sample. In some embodiments, the sample is a blood sample. As used herein the term "blood sample" means any blood sample derived from the subject. Collections of blood samples can be performed by methods well known to those skilled in the art. For example, the subject's blood can be drawn by trained medical personnel directly into anti-coagulants such as citrate and EDTA. The whole blood can be separated into the plasma portion, the cells, and platelets portion by refrigerated centrifugation at 3500 X G for 2 minutes. After centrifugation, the supernatant is the plasma.

More particularly, a further object of the present invention relates to a method for detecting the presence of infectious particles of hepatitis E virus in a sample comprising contacting the sample with the antibodies of the present invention under conditions that allow an immunocomplex of the antibody and the infectious particles to form wherein detection of the immunocomplex indicates the presence of the infectious particles in the sample.

In some embodiments, the detecting methods of the present invention may further comprises contacting the sample with antibodies recognizing the ORF2g and/or ORF2c proteins.

The detecting methods of the present invention are particularly suitable for diagnosing acute HEV infection, recent HEV infection, chronic HEV infection, weak active HEV infection or cleared HEV infection.

Assays and conditions for the detection of immunocomplexes are known to those of skill in the art. Such assays include, for example, competition assays, direct reaction assays sandwich- type assays and immunoassays (e.g. ELISA). The assays may be quantitative or qualitative. There are a number of different conventional assays for detecting formation of an antibody-peptide complex comprising a protein of the present invention. For example, the detecting step can comprise performing an ELISA assay, performing a lateral flow immunoassay, performing an agglutination assay, analyzing the sample in an analytical rotor, or analyzing the sample with an electrochemical, optical, or opto-electronic sensor. These different assays are well-known to those skilled in the art.

For example, any of a number of variations of the sandwich assay technique may be used to perform an immunoassay. Briefly, in a typical sandwich assay, a first antibody of the present invention is immobilized on a solid surface and the sample to be tested is brought into contact with the immobilized antibody for a time and under conditions allowing formation of the immunocomplex. Following incubation, a second antibody of the present invention that is labeled with a detectable moiety is added and incubated under conditions allowing the formation of a ternary complex between any immunocomplex and the labeled antibody. Any unbound material is washed away, and the presence of polypeptide in the sample is determined by observation/detection of the signal directly or indirectly produced by the detectable moiety. Detection may be either qualitative or quantitative. Methods for labeling biological molecules such as antibodies are well-known in the art (see, for example, "Affinity Techniques. Enzyme Purification: Part B", Methods in EnzymoL, 1974, Vol. 34, W.B. Jakoby and M. Wilneck (Eds.), Academic Press: New York, NY; and M. Wilchek and E.A. Bayer, Anal. Biochem., 1988, 171 : 1-32). The most commonly used detectable moieties in immunoassays are enzymes and fluorophores. In the case of an enzyme immunoassay (EIA or ELISA), an enzyme such as horseradish peroxidase, glucose oxidase, beta-galactosidase, alkaline phosphatase, and the like, is conjugated to the second antibody, generally by means of glutaraldehyde or periodate. The substrates to be used with the specific enzymes are generally chosen for the production of a detectable color change, upon hydrolysis of the corresponding enzyme. In the case of immunofluorescence, the second antibody is chemically coupled to a fluorescent moiety without alteration of its binding capacity. After binding of the fiuorescently labeled antibody to the immunocomplex and removal of any unbound material, the fluorescent signal generated by the fluorescent moiety is detected, and optionally quantified. Alternatively, the second antibody may be labeled with a radioisotope, a chemiluminescent moiety, or a bio luminescent moiety. In some embodiments, the assay utilizes a solid phase or substrate to which the antibody of the present invention is directly or indirectly attached. Accordingly in some embodiments, the antibody of the present invention is attached to or immobilized on a substrate, such as a solid or semi-solid support. The attachment can be covalent or non-covalent, and can be facilitated by a moiety associated with the protein that enables covalent or non-covalent binding, such as a moiety that has a high affinity to a component attached to the carrier, support or surface. In some embodiments, the substrate is a bead, such as a colloidal particle (e.g., a colloidal nanoparticle made from gold, silver, platinum, copper, metal composites, other soft metals, core-shell structure particles, or hollow gold nanospheres) or other type of particle (e.g., a magnetic bead or a particle or nanoparticle comprising silica, latex, polystyrene, polycarbonate, polyacrylate, or PVDF). Such particles can comprise a label (e.g., a colorimetric, chemiluminescent, or fluorescent label) and can be useful for visualizing the location of the proteins during immunoassays. In some embodiments, the substrate is a dot blot or a flow path in a lateral flow immunoassay device. For example, the antibody of the present invention can be attached or immobilized on a porous membrane, such as a PVDF membrane (e.g., an Immobilon^{™} membrane), a nitrocellulose membrane, polyethylene membrane, nylon membrane, or a similar type of membrane. In some embodiments, the substrate is a flow path in an analytical rotor. In some embodiments, the substrate is a tube or a well, such as a well in a plate (e.g., a microtiter plate) suitable for use in an ELISA assay. Such substrates can comprise glass, cellulose-based materials, thermoplastic polymers, such as polyethylene, polypropylene, or polyester, sintered structures composed of particulate materials (e.g., glass or various thermoplastic polymers), or cast membrane film composed of nitrocellulose, nylon, polysulfone, or the like. A substrate can be sintered, fine particles of polyethylene, commonly known as porous polyethylene, for example, 0.2-15 micron porous polyethylene from Chromex Corporation (Albuquerque, N. Mex.). All of these substrate materials can be used in suitable shapes, such as films, sheets, or plates, or they may be coated onto or bonded or laminated to appropriate inert carriers, such as paper, glass, plastic films, or fabrics. Suitable methods for immobilizing peptides on solid phases include ionic, hydrophobic, covalent interactions and the like.

A further object of the present invention relates to a kit or device for identifying the presence of infectious hepatitis E viral particles in a sample comprising at least one antibody of the present invention (immobilized or not on a solid support as described above). In some embodiments, the kit can include a second antibody of the present invention which produces a detectable signal. In some embodiments, the kit further comprises antibodies having specificity for ORF2g and/or ORF2c proteins. Examples of kits include but are not limited to ELISA assay kits, and kits comprising test strips and dipsticks. In some embodiments, the kits described herein further comprise reference values of the levels of the protein or infectious particles. The reference values are typically average levels in samples from a population of healthy individuals. In some embodiments, the kits described herein further comprise at least one sample collection container for sample collection. Collection devices and container include but are not limited to syringes, lancets, BD VACUTAINER^{®} blood collection tubes. In some embodiments, the kits described herein further comprise instructions for using the kit and interpretation of results.

The invention will be further illustrated by the following figures and examples. However, these examples and figures should not be interpreted in any way as limiting the scope of the present invention.

### FIGURES:

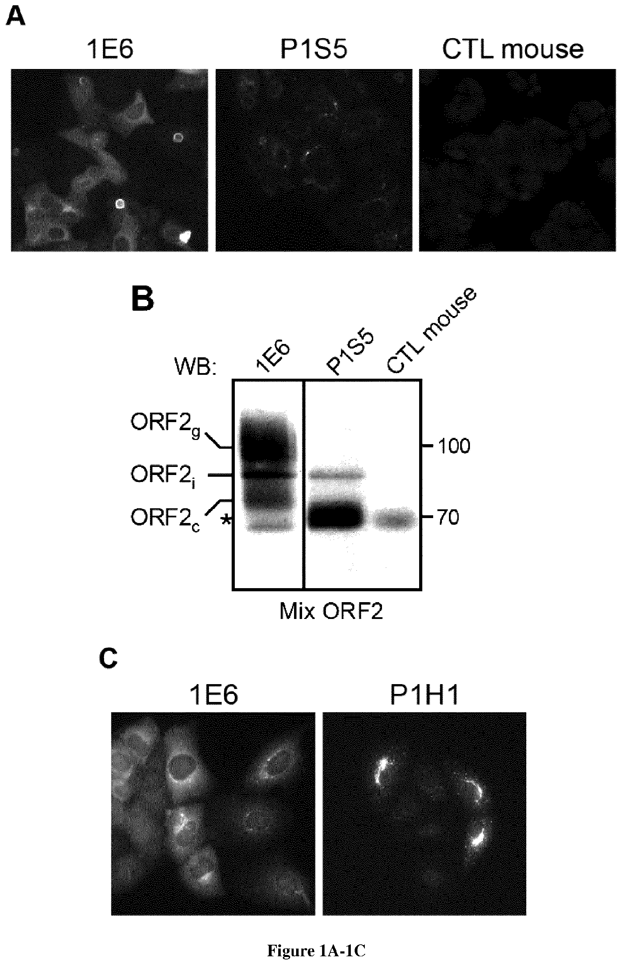

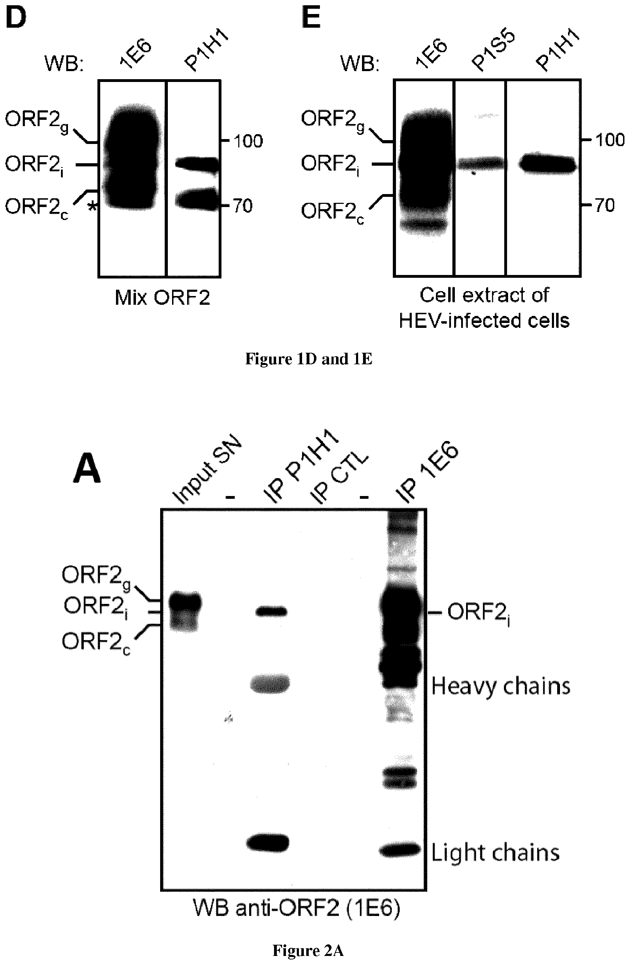

**Figure 1****: Generation of specific antibodies directed against the ORF2i protein using the peptide GQPSGRRRGRRSGG (SEQ ID NO:4).** Reactivity and specificity of P1S5 and control (CTL) mouse sera and 1E6 antibody were analyzed (**A**) by immunofluorescence on fixed HEV-infected cells and (**B**) by western blotting on a mixture of ORF2 proteins (ORF2i and ORF2g/ORF2c). The asterisk indicates a non-specific band. Reactivity and specificity of the P1H1 hybridoma supernatant and 1E6 antibody were analyzed (**C**) by immunofluorescence on fixed HEV-infected cells and by western blotting on a mixture of ORF2 proteins (**D**) or an extract of HEV-infected cells (**E**).

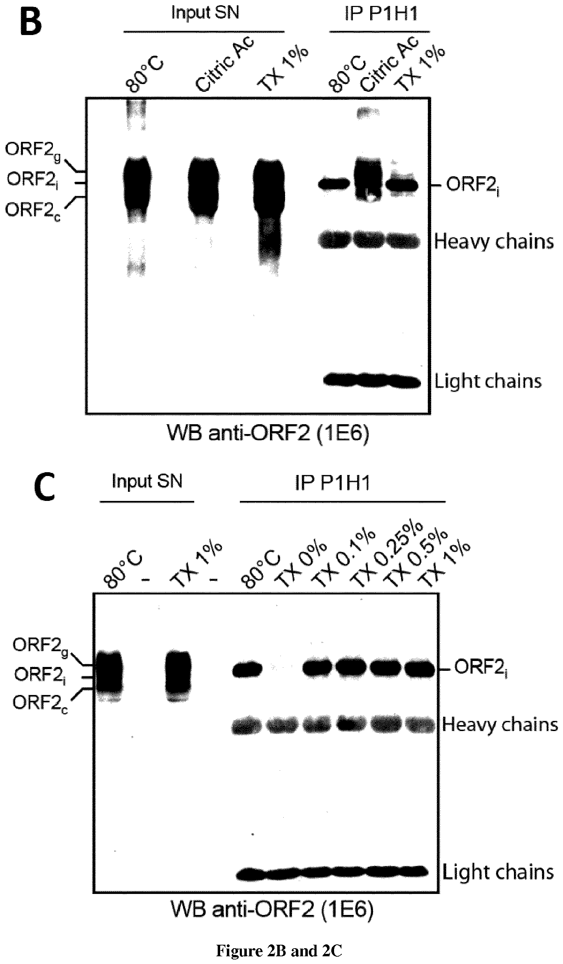

**Figure 2****: Immunoprecipitation of detergent-treated HEV particles with P1H1 antibody. (A-C)** Sensitivity and specificity of IP P1H1 were analyzed by western blot using the 1E6 antibody.

### RESULTS: Generation of specific antibodies directed against the ORF2i protein using the peptide GQPSGRRRGRRSGG (SEQ ID NO:4)

The N-terminal portion is present only in the ORF2i form and not in the ORF2g form nor the ORF2c form. Therefore said portion can represent the best strategy for obtaining highly specific antibodies of the ORF2i form. In order to verify the specificity between strains / genotypes, sequence alignment only between amino acids (a.a) 1 to 50 was carried out (data not shown). The prediction of secondary structure and absence of glycosylation, as well as prediction of immunogenicity, hydrophilicity, and accessibility were performed to define the best sequence for the immunogen. Finally, the peptide GQPSGRRRGRRSGG (SEQ ID NO:4) was selected for the immunization. In order to make this sequence more immunogenic during murine immunizations, a coupling to the protein carrier KLH was carried out via a maleimide function by adding a cysteine at the C-terminal position. Immunization was performed according a routine protocol. Five mice were immunized at least three times at three weeks intervals with the peptide (SEQ ID NO:4). Freund's complete and incomplete adjuvants were used during immunisation. The animals were immunized by subcutaneous and intraperitoneal routes. Ten days after the third immunisation, mice have been bleeded and their sera tested for immunoreactivity. Sera were first assayed by indirect ELISA on plates coated with the peptide (SEQ ID NO:4) (data not shown). Their reactivity was analysed by immunofluorescence (IF) on fixed HEV-infected cells (**Figure 1A**). The 1E6 monoclonal antibody (antibody registry #AB-827236), which recognizes the three forms of ORF2 proteins (Montpellier et al., Gastroenterology, 2018*),* was used as a positive control. The serum of a PBS-immunized mouse was used as a negative control (CTL mouse). As shown in **Figure 1A**, serum of the P1S5 mouse showed a specific reactivity in IF. Specificity of the P1S5 serum was next analysed in western blotting experiments with a mixture of ORF2 proteins (ORF2i and ORF2g/ORF2c) as antigens (**Figure 1B**). The P1S5 serum showed a highly specific recognition of the ORF2i protein, with no cross-reaction with the ORF2g/c proteins, as compared to the 1E6 antibody that recognizes the three forms.

After a final boost, the P1S5 mouse was sacrificed. Lymphocytes were isolated from the spleen and fused with a myeloma cell line using polyethylene glycol to form a hydridoma. Following fusion, cells were placed in media permissive for growth of hybridomas. Following culture of the hybridomas, cell supernatants were screened by immunofluorescence. As shown in **Figure 1C**, the P1H1 hybridoma supernatant displayed an ORF2i-specific staining. Specificity of the P1H1 clone was next analysed in western blotting experiments with a mixture of ORF2 proteins (ORF2i and ORF2g/ORF2c) (**Figure 1D**) or an extract of HEV-infected cells (**Figure 1E**), as antigens. The P1H1 clone showed a highly specific recognition of the ORF2i protein, with no cross-reaction with the ORF2g/c proteins, as compared to the 1E6 antibody that recognizes the three forms.

The P1H1 clone was next amplified and purified. The purified P1H1 antibody was used in immunoprecipitation (IP) assays (**Figure 2**). Heat-inactivated (20 min at 80°C) supernatant of HEV-producing cells (SN) was incubated with P1H1 antibody or 1E6 antibody and then with Protein-G sepharose beads. Immunoprecipitated ORF2 proteins were analyzed by western blot using the 1E6 antibody. The IP P1H1 showed a highly specific recognition of the ORF2i protein, with no cross-reaction with the ORF2g/c proteins and no background (**Figure 2A**), as compared to the IP 1E6 that displayed the three forms of ORF2 and multiple non specific bands. SN incubated only with Protein-G beads was used as a negative control (IP CTL). These results indicate that the P1H1 antibody is able to immunoprecipitate heat-denatured HEV particles.

SN was then either heat-inactivated for 20 min at 80°C, or treated for 20 min with 3% citric acid and then buffered with 1M Tris pH 8, or treated for 30 min with 1% Triton X-100 (**Figure 2B**). SN were next subjected to IP with the P1H1 antibody. Immunoprecipitated ORF2 proteins were analyzed by western blot using the 1E6 antibody. The IP P1H1 on citric acid-treated SN displayed only a faint band of ORF2i protein and non-specific bands. In contrast, as for the IP P1H1 on heat-inactivated SN, the IP P1H1 on Triton X-100-treated SN showed a highly specific recognition of the ORF2i protein, with no cross-reaction with the ORF2g/c proteins and no background (**Figure 2B**). SN was then treated for 30 min with 0.1%, 0.25%, 0.5% or 1% Triton X-100 before IP with P1H1 (**Figure 2C**). Untreated SN (TX 0%) and heat-denatured SN (80°C) were used as negative and positive controls, respectively. Immunoprecipitated ORF2 proteins were analyzed by western blot using the 1E6 antibody. In all samples treated with Triton X-100 (0.1 to 1%), the IP P1H1 showed a highly sensitive and specific recognition of the ORF2i protein, with no cross-reaction with the ORF2g/c proteins and no background (**Figure 2C**). Taken together, these results indicate that the P1H1 antibody is able to specifically immunoprecipitate detergent-treated HEV particles.

Together, these results indicate that antibodies specifically directed against the ORF2i polypeptides (SEQ ID NO:4) can be produced. Such antibodies will be very suitable for determining presence of infectious particles of hepatitis E virus in a sample. More particularly, detection of the ORF2i polypeptide of the present invention is suitable for diagnosing hepatitis E virus infection in a subject.

### REFERENCES:

Throughout this application, various references describe the state of the art to which this invention pertains.

## Claims

1. An antibody which binds to the ORF2i protein of hepatitis E virus and wherein said antibody does not bind to the ORF2g protein nor to the ORF2c protein of hepatitis E virus, and wherein the antibody binds to an epitope comprising the amino acid sequence as set forth in SEQ ID NO: 4.

2. The antibody of claim 1 which is a polyclonal antibody or a monoclonal antibody.

3. The antibody of claim 1 which is a Fab', Fab, F(ab')2, scFv or a single domain antibody.

4. The antibody of claim 1 which is conjugated with a detectable label.

5. The antibody of claim 4 wherein the detectable label is a radioisotope, a fluorescent label, a chemiluminescent label, an enzyme label, or a bio luminescent label.

6. The antibody of claim 4 wherein the label is selected from the group consisting of β-galactosidase, glucose oxidase, peroxidase (e.g. horseradish peroxidase) and alkaline phosphatase.

7. The antibody of claim 1 which is attached to or immobilized on a substrate, such as a solid or semi-solid support.

8. Use of the antibody of claim 1 for determining presence of infectious particles of hepatitis E virus in a sample.

9. A method for detecting the presence of the ORF2i protein in a sample comprising contacting the sample with the antibody of claim 1 under conditions that allow an immunocomplex of the protein and antibody to form wherein detection of the immunocomplex indicates the presence of the ORF2i protein in the sample.

10. A method for detecting the presence of infectious particles of hepatitis E virus in a sample comprising contacting the sample with the antibody of claim 1 under conditions that allow an immunocomplex of the antibody and the infectious particles to form wherein detection of the immunocomplex indicates the presence of the infectious particles in the sample.

11. The method of claim 9 or 10 wherein the sample is selected from the group consisting of faeces, blood, ascites; urine; saliva; sweat; milk; synovial fluid; peritoneal fluid; amniotic fluid; cerebrospinal fluid; lymph fluid; lung embolism; and pericardial fluid.

12. Use of the antibody of claim 1 for diagnosing acute HEV infection, recent HEV infection, chronic HEV infection, weak active HEV infection or cleared HEV infection.

13. A kit or device for identifying the presence of infectious hepatitis E viral particles in a sample comprising at least one antibody of claim 1 immobilized or not on a solid support wherein the device is a flow immunoassay device.

## Patentansprüche

1. Antikörper, der sich an das 0RF2i-Protein des Hepatitis-E-Virus bindet und wobei sich der Antikörper weder an das ORF2g-Protein noch an das ORF2c-Protein des Hepatitis-E-Virus bindet, und wobei sich der Antikörper an ein Epitop bindet, das die Aminosäuresequenz wie in SEQ ID NO: 4 dargelegt umfasst.

2. Antikörper nach Anspruch 1, der ein polyklonaler Antikörper oder ein monoklonaler Antikörper ist.

3. Antikörper nach Anspruch 1, der ein Fab', Fab, F(ab')2, scFv oder ein Einzeldomänenantikörper ist.

4. Antikörper nach Anspruch 1, der mit einem nachweisbaren Marker konjugiert ist.

5. Antikörper nach Anspruch 4, wobei der nachweisbare Marker ein Radioisotop, ein fluoreszierender Marker, ein chemilumineszierender Marker, ein Enzymmarker oder ein biolumineszierender Marker ist.

6. Antikörper nach Anspruch 4, wobei der Marker ausgewählt ist aus der Gruppe bestehend aus β-Galactosidase, Glucoseoxidase, Peroxidase (z. B. Meerrettichperoxidase) und alkalischer Phosphatase.

7. Antikörper nach Anspruch 1, der an ein Substrat, wie einen festen oder halbfesten Träger, gebunden oder darauf immobilisiert ist.

8. Verwendung des Antikörpers nach Anspruch 1 zum Bestimmen des Vorhandenseins von infektiösen Partikeln des Hepatitis-E-Virus in einer Probe.

9. Verfahren zum Nachweisen des Vorhandenseins des 0RF2i-Proteins in einer Probe, umfassend das In-Kontakt-bringen der Probe mit dem Antikörper nach Anspruch 1 unter Bedingungen, die die Bildung eines Immunkomplexes aus dem Protein und dem Antikörper ermöglichen, wobei ein Nachweis des Immunkomplexes das Vorhandensein des 0RF2i-Proteins in der Probe anzeigt.

10. Verfahren zum Nachweisen des Vorhandenseins von infektiösen Partikeln des Hepatitis-E-Virus in einer Probe, umfassend das In-Kontakt-bringen der Probe mit dem Antikörper nach Anspruch 1 unter Bedingungen, die die Bildung eines Immunkomplexes aus dem Antikörper und den infektiösen Partikeln ermöglichen, wobei ein Nachweis des Immunkomplexes das Vorhandensein der infektiösen Partikel in der Probe anzeigt.

11. Verfahren nach Anspruch 9 oder 10, wobei die Probe ausgewählt ist aus der Gruppe bestehend aus Fäkalien, Blut, Aszites; Urin; Speichel; Schweiß; Milch; Gelenkflüssigkeit; Peritonealflüssigkeit; Fruchtwasser; Liquor cerebrospinalis; Lymphflüssigkeit; Lungenembolie; und Perikardflüssigkeit.

12. Verwendung des Antikörpers nach Anspruch 1 zum Diagnostizieren einer akuten HEV-Infektion, einer frischen HEV-Infektion, einer chronischen HEV-Infektion, einer schwachen aktiven HEV-Infektion oder einer beseitigten HEV-Infektion.

13. Kit oder Vorrichtung zum Identifizieren des Vorhandenseins von infektiösen viralen Hepatitis-E-Partikeln in einer Probe, umfassend mindestens einen Antikörper nach Anspruch 1, der auf einem festen Träger immobilisiert ist oder nicht, wobei die Vorrichtung eine Durchfluss-Immunassay-Vorrichtung ist.

## Revendications

1. Anticorps qui se lie à la protéine ORF2i du virus de l'hépatite E et dans lequel ledit anticorps ne se lie ni à la protéine ORF2g ni à la protéine ORF2c du virus de l'hépatite E, et dans lequel l'anticorps se lie à un épitope comprenant la séquence d'acides aminés telle qu'indiquée dans SEQ ID NO : 4.

2. Anticorps selon la revendication 1 qui est un anticorps polyclonal ou un anticorps monoclonal.

3. Anticorps selon la revendication 1 qui est un Fab', un Fab, un F(ab')2, un scFv ou un anticorps à domaine unique.

4. Anticorps selon la revendication 1 qui est conjugué à un marqueur détectable.

5. Anticorps selon la revendication 4, dans lequel le marqueur détectable est un radioisotope, un marqueur fluorescent, un marqueur chimio-luminescent, un marqueur enzymatique, ou un marqueur bioluminescent.

6. Anticorps selon la revendication 4, dans lequel le marqueur est sélectionné parmi le groupe consistant en la β-galactosidase, la glucose oxydase, la peroxydase (p. ex. la peroxydase de raifort) et la phosphatase alcaline.

7. Anticorps selon la revendication 1 qui est fixé à ou immobilisé sur un substrat, tel qu'un support solide ou semi-solide.

8. Utilisation de l'anticorps selon la revendication 1 pour déterminer la présence de particules infectieuses du virus de l'hépatite E dans un échantillon.

9. Procédé de détection de la présence de la protéine ORF2i dans un échantillon comprenant la mise en contact de l'échantillon avec l'anticorps selon la revendication 1 dans des conditions qui permettent à un immunocomplexe de la protéine et de l'anticorps de se former, dans lequel la détection de l'immunocomplexe indique la présence de la protéine ORF2i dans l'échantillon.

10. Procédé de détection de la présence de particules infectieuses du virus de l'hépatite E dans un échantillon comprenant la mise en contact de l'échantillon avec l'anticorps selon la revendication 1 dans des conditions qui permettent à un immunocomplexe de l'anticorps et des particules infectieuses de se former, dans lequel la détection de l'immunocomplexe indique la présence des particules infectieuses dans l'échantillon.

11. Procédé selon la revendication 9 ou 10, dans lequel l'échantillon est sélectionné parmi le groupe consistant en les selles, le sang, l'ascite ; l'urine ; la salive ; la sueur ; le lait ; le liquide synovial ; le liquide péritonéal; le liquide amniotique ; le liquide céphalorachidien; le liquide lymphatique ; l'embolie pulmonaire ; ; et le liquide péricardique.

12. Utilisation de l'anticorps selon la revendication 1 pour diagnostiquer une infection aiguë par le VHE, une infection récente par le VHE, une infection chronique par le VHE, une légère infection active par le VHE ou une infection par le VHE guérie.

13. Kit ou dispositif pour identifier la présence de particules virales infectieuses de l'hépatite E dans un échantillon comprenant au moins un anticorps selon la revendication 1 immobilisé ou non sur un support solide dans lequel le dispositif est un dispositif d'immuno-essai à écoulement.
